# FASCICULE DE BREVET EUROPEEN

(11) **EP 2 403 474 B1**
(45) Date de publication et mention de la délivrance du brevet: **11.01.2017**
(21) Numéro de dépôt: 10710868.0
(22) Date de dépôt: 04.03.2010
(51) Int. Cl.: A61Q 19/08, A61K 8/29, A61K 8/67, A61K 8/02, A61K 8/73, A61K 8/11, A61K 8/64

(54) **COMPOSITION COSMÉTIQUE POUR LUTTER CONTRE LE VIEILLISSEMENT DE LA PEAU, CONSTITUÉE ET DISTRIBUÉE SOUS LA FORME D'UNE POUDRE SE TRANSFORMANT EN CRÈME LORS DE SON APPLICATION**
Anti-hautalterung Kosmetische Zusammensetzung in Form eines Pulvers verwandelnd sich in eine Creme während des Auftragens
COSMETIC COMPOSITION FOR COMBATING SKIN AGEING, FORMULLATED AND DISPENSED IN THE FORM OF A POWDER TURNING INTO A CREAM UPON APPLICATION

(30) Priorité: 04.03.2009 FR 0900999
(43) Date de publication de la demande: 11.01.2012
(73) Titulaire: CCI R & D, 27400 Acquigny (FR)
(72) Inventeur: LE BASTARD, Patricia, F-27700 Guiseniers (FR)
(74) Mandataire: Degret, Jacques
(86) Numéro de dépôt international: PCT/FR2010/000188
(87) Numéro de publication internationale: WO 2010/100350

(56) Documents cités:
- WO-A1-00/28961
- FR-A1- 2 844 448
- US-A- 6 123 930
- US-A1- 2003 199 576
- US-A1- 2006 292 108
- US-B1- 7 306 810
- Dr Sebagh: "PURE VITAMIN C POWDER CREAM", Internet citation, 12 janvier 2008 (2008-01-12), XP002561837, Extrait de l'Internet: URL:http://web.archive.org/web/20080112001 314/www.drsebagh.com/index-2.html [extrait le 2010-01-04]
- Mintel: "High Definition Body Lift", , février 2009 (2009-02), XP002612720, Extrait de l'Internet: URL:http://www.gnpd.com/sinatra/gnpd/searc h_results/&item_id=1061655 [extrait le 2010-12-06]

## Description

La présente invention a pour objet une composition pour le traitement cosmétique des imperfections ou affections de la peau et elle se rapporte plus particulièrement à une composition cosmétique pour lutter contre le vieillissement de la peau et comprenant à cette fin un produit actif apte à apporter sur la peau des molécules capables d'aider les cellules à se défendre tant contre les effets de l'âge que contre l'exposition à la lumière, notamment ultraviolette, qui entraîne la formation en excès de radicaux libres photo-induits tels les radicaux peroxydes, superoxydes et hydroxyles.

On sait que, au cours du processus de vieillissement, il apparaît différents signes caractéristiques sur la peau, qui se traduisent notamment par une modification de la structure et des fonctions cutanées. Les signes de vieillissement existants, plus spécialement photo-induits, sont notamment les rides et les ridules dont l'apparition augmente avec l'âge et qui se traduisent par une dépression ou par des sillons à la surface de la peau. D'autres signes de vieillissement cutané, qui résultent d'un dysfonctionnement des principaux mécanismes biologiques intervenant au niveau de la peau, se traduisent par exemple par une desquamation, par une modification du teint, en l'occurrence un ternissement, et par une rugosité et une sécheresse en surface.

L'une des molécules bien connues capable de lutter efficacement contre la formation de radicaux libres oxygénés, et donc de renforcer les défenses du tissu cutané contre les agressions extérieures tels la pollution et les rayonnements ultraviolets, est l'acide ascorbique ou vitamine C réputé pour ses propriétés antioxydantes. Un autre avantage de l'acide ascorbique est qu'il stimule la synthèse du tissu conjonctif, plus particulièrement la biosynthèse du collagène de la peau.

On a pu également constater que l'acide ascorbique catalyse l'hydroxylation de la proline et de la lysine en hydroxyproline et en hydroxylysine, constituants normaux du collagène. Or, on sait que le collagène forme 30% de la totalité des protéines de l'organisme, qu'il entre dans la composition de la peau et qu'il exerce une action efficace sur l'élasticité et la fermeté de la peau.

On connaît par ailleurs des compositions cosmétiques qui sont réalisées et distribuées sous la forme d'une poudre, laquelle se transforme en crème au moment où on l'applique sur la peau sous le simple effet de la chaleur de la peau combinée à une contrainte mécanique (par exemple le frottement du doigt).

Ces compositions cosmétiques constituées par une poudre solide comprennent une phase aqueuse sous forme encapsulée ou immobilisée à la surface d'un solide et associée à un agent gélifiant de ladite phase aqueuse, généralement un amidon, et à des particules d'oxyde métallique, généralement des particules à base d'oxyde de titane, présentant des propriétés de surface leur conférant une affinité faible vis-à-vis de la phase aqueuse.

Ces compositions cosmétiques ayant la forme d'une poudre et se transformant en crème lors de leur application peuvent ou non comprendre un principe actif cosmétique.

Ainsi, il a déjà été proposé des compositions parfumées, des compositions antisudorantes, des compositions hydratantes et des compositions amincissantes.

Cela étant, il n'a jamais été proposé de compositions cosmétiques du type précité qui soient actives contre le vieillissement, c'est-à-dire des compositions cosmétiques encore appelées "anti-âge".

En effet, il n'a pas été possible jusqu'à aujourd'hui d'introduire de l'acide ascorbique en tant qu'agent actif dans ce type de compositions car l'acide ascorbique, en raison de sa structure chimique et de ses propriétés réductrices, est particulièrement sensible à certains paramètres de l'environnement tels que la lumière, l'oxygène (il s'oxyde facilement, surtout en milieu alcalin) et l'humidité (il est très sensible en milieu aqueux), d'où une instabilité certaine.

Les rares produits cosmétiques contenant de l'acide ascorbique que l'on connaisse sont soit des poudres se transformant en liquides, ainsi qu'il est par exemple décrit dans la demande de brevet US-2003/0199576 publiée le 23 octobre 2003, dans lesquelles le taux d'acide ascorbique est particulièrement élevé, soit des crèmes dans lesquelles le taux d'acide ascorbique est également très élevé et, pour que de telles émulsions demeurent stables, ne peut dès lors être incorporé que sous la forme de l'un de ses sels (phosphate, oléate ou palmitate). Le brevet US-7.306.810 publié le 11 décembre 2007 décrit de tels types de crèmes.

Lorsque l'acide ascorbique est mélangé à une composition cosmétique du premier type précité, c'est-à-dire distribuée sous la forme d'une poudre apte à se transformer en crème au moment où on l'applique sur la peau, ladite poudre se casse, il y a rupture du système et, à la place de la poudre, on obtient un amalgame pâteux.

En outre, l'acide ascorbique perd ses propriétés et se dégrade rapidement, ce qui va à l'encontre de l'efficacité recherchée puisque, au moment de son utilisation, la composition cosmétique ne contiendrait alors plus aucun produit actif contre le vieillissement.

Quant à la solution préconisée selon le document US-2003/0199576, outre le fait que la poudre fabriquée se transforme en liquide et non pas en crème (le taux d'acide ascorbique utilisé est en effet si important qu'il rend impossible l'obtention d'une émulsion), elle a l'inconvénient supplémentaire d'imposer une opération de séchage, donc de déshydratation, pour que l'acide ascorbique puisse rester stable à de tels taux aussi élevés.

Or, en matière de produits cosmétiques, l'obtention finale d'une crème de préférence à un liquide est essentielle. La transformation du produit de base, ici une poudre, en « liquide » ne permet pas une application aisée et confortable d'un tel produit cosmétique sur le visage de son utilisateur/utilisatrice alors que la transformation de la poudre de base en « crème » est considérée par tout utilisateur/utilisatrice comme un élément de confort pour l'application du produit cosmétique.

Il semble d'ailleurs que le procédé décrit dans le document US-2003/0199576 concerne davantage la conservation de l'acide ascorbique à fort taux en tant que matière première plutôt qu'une application directe sur le visage, ce qui pourtant serait le but d'un produit fini cosmétique, vu le taux de 20% d'acide ascorbique incorporé.

La présente invention remédie aux inconvénients exposés ci-dessus et elle propose une composition cosmétique sous forme de poudre qui peut être stockée à température ordinaire (c'est-à-dire inférieure à 35°C) et transportée sous cette forme, laquelle poudre se transforme en crème au moment de son application et libère alors de l'acide ascorbique pur, actif contre les méfaits du vieillissement de la peau.

A cette fin, la composition cosmétique selon l'invention est remarquable en ce que sa phase aqueuse comprend de l'acide ascorbique, à titre de premier agent actif, dans la plage en poids/poids de 0,01% à 0,20%, un second agent actif, choisi parmi une protéine végétale ou la caféine, et un agent d'ajustement de son pH entre 7,7 et 8,2.

Dans un mode de réalisation préféré, la composition précitée comprend entre 0,01% et 0,20% en poids/poids d'acide ascorbique, et de préférence environ 0,10% de cet acide dans son application aux soins du visage et d'environ 0,01 à 0,05% dans son application aux contours des yeux pour réduire, voire éliminer, les poches et les cernes.

L'agent d'ajustement du pH est avantageusement choisi comme étant la triéthanolamine, et le rapport en poids/poids de l'acide ascorbique et de la triéthanolamine varie alors entre environ 1:1,5 et environ 1:3.

Dans une variante particulièrement avantageuse d'application aux soins du visage, le second agent actif contenu dans la phase aqueuse de la composition cosmétique est un agent actif dit "coup d'éclat", lequel agent actif est une protéine végétale qui facilite le drainage dans les vaisseaux sanguins et qui vient renforcer la propriété d'exfoliant de l'acide ascorbique.

Par agent actif dit "coup d'éclat", il faut comprendre un agent qui stimule le facteur de croissance vasculaire, c'est-à-dire qui améliore la microcirculation cutanée et l'oxygénation de la peau en stimulant la synthèse du facteur de croissance vasculaire. La lumière qui traverse l'épiderme ainsi traité se charge en lumière rouge au niveau des capillaires sanguins, procurant un effet coloré, légèrement rosé. L'expression agent actif "coup d'éclat" est celle qui est la plus couramment employée dans le domaine des cosmétiques, mais l'on emploie aussi des expressions tels que "actif révélateur d'éclat" ou encore "effet "anti-teint terne" ou encore "enlumineur de teint".

L'agent actif "coup d'éclat" contient avantageusement de l'octénylsuccinate de protéine de lupin blanc doux hydrolysée, de préférence dans la proportion de 0,30% à 0,90%, et de préférence encore dans la proportion de 0,40% à 0,60%.

Dans une variante d'application aux contours des yeux, pour réduire, voire éliminer les poches et les cernes, le second agent actif contenu dans la phase aqueuse de la composition cosmétique est de la caféine, dans la proportion de 0,01 à 1%.

De préférence, l'agent gélifiant est de l'amidon carboxyméthylé sodique, dans la proportion de 0,10 à 15%.

De préférence également, la phase aqueuse de la composition cosmétique selon l'invention comprendra du phénoxyéthanol, idéalement dans la proportion de 1% environ.

La composition selon l'invention comprendra également avantageusement de la glycérine, à titre d'agent humectant, dans la proportion d'environ 3%.

Les caractéristiques et avantages de la présente invention apparaîtront de façon encore plus évidente au vu des exemples illustratifs détaillés ci-après pour des compositions cosmétiques, d'une part, et pour leur mode opératoire, d'autre part.

Il est tout d'abord donné ci-après un exemple général et deux exemples préférentiels de composition cosmétique pour lutter contre le vieillissement de la peau du visage, constituée et distribuée sous la forme d'une poudre se transformant spontanément en crème lors de son application sur le visage.

Dans ces exemples, et dans ceux qui suivront, les pourcentages des différents constituants sont toujours définis en poids exprimés par rapport au poids total de la composition concernée.

### Exemples de Composition pour les soins du visage :

### A- Exemple général

### (I) Phase liquide aqueuse :

| | |
|---|---|
| - Eau osmosée (eau de ville déminéralisée à travers un osmoseur qui arrête les bactéries) | 50 à 90% |
| - Actifs hydrosolubles | 1 à 15% |
| - Acide ascorbique | 0,01 à 0,20% |
| - Extrait aqueux protéiné de lupin | 0,10 à 5% |
| - Triéthanolamine | 0,05 à 0,30% |
| - Conservateur(s) | 0 à 1% |

### (II) Poudre solide :

| | |
|---|---|
| - Agent(s) gélifiant(s) | 0,1 à 15% |
| - Emulsifiant(s) | 0 à 10% |
| - Actif(s) hydrosoluble(s) ou liposoluble(s) | 0 à 20% |
| - Particules traitées ou non en surface | 0 à 20% |
| - Liant(s) | 0 à 10% |
| - Conservateur(s) | 0 à 1% |

### B- Premier exemple préférentiel

### (I) Phase liquide aqueuse :

| | |
|---|---|
| - Eau osmosée (eau de ville déminéralisée à travers un osmoseur qui arrête les bactéries) | 75,98% |
| - Phénoxyéthanol (commercialisé par Laserson) | 0,97% |
| - Eclaline 2 (commercialisée par Silab et constituée de 77,50% d'eau, de 21% d'octényl-succinate de protéine de lupin blanc doux hydrolysée et de 1,05% de phénoxyéthanol) | 2,00% |
| - Acide ascorbique à 100% (commercialisé par Fagron) | 0,10% |
| - Glycérine à 100% (commercialisée par A.M.I.) | 3,00% |
| - Triéthanolamine à 100% (commercialisée par Laboratoire Prod'hyg) | 0,15% |

Dans cette phase aqueuse, le phénoxyéthanol est un agent conservateur, la glycérine est un agent humectant et la triéthanolamine est un agent d'ajustement du pH.

### (II) Poudre solide :

| | |
|---|---|
| - Amidon carboxyméthylé sodique à 100% | 3,80% |
| - Mica | 2,38% |
| - Talc + Silicone | 4,55% |
| - Triéthoxycaprylylsilane | 0,07% |
| - Méthylméthacrylate réticulé en billes à 100% | 3,00% |
| - Dioxyde de titane traité par des triméthoxycaprylylsilanes et des triéthoxycaprylylsilanes | 3,00% |
| - Fumarate de stéaryl sodium à 100% | 1,00% |

formulation dans laquelle l'amidon carboxyméthylé sodique est l'agent gélifiant et le dioxyde de titane, en ce qu'il est traité par des groupements silanes, à savoir des triméthoxycaprylylsilanes et des triéthoxycaprylylsilanes, possède une surface de nature hydrophobe présentant en conséquence des propriétés de surface capables de lui conférer une affinité faible vis-à-vis de la phase aqueuse.

### C- Deuxième exemple préférentiel

### (I) Phase liquide aqueuse :

| | |
|---|---|
| - Eau osmosée | 71,49% |
| - Glycérine | 3,00% |
| - Polyoxyéthylène glycol | 5,50% |
| - Acide ascorbique | 0,10% |
| - Eclaline 2 (commercialisée par Silab et constituée de 77,50% d'eau, de 21% d'octénylsuccinate de protéine de lupin blanc doux hydrolysée et de 1,05% de phénoxyéthanol) | 2,00% |
| - Triéthanolamine à 100% | 0,20% |
| - Méthylisothiazolinone | 0,10% |

formulation dans laquelle le méthylisothiazolinone est un agent conservateur, commercialisé par la société Thor sous la marque Microcare MT, tandis que les deux agents actifs hydrosolubles que sont la glycérine et le polyoxyéthylène glycol (commercialisé par la société Croda sous la marque Renex PEG-4000) constituent des agents humectants.

### (II) Poudre solide :

| | |
|---|---|
| - Amidon carboxyméthylé sodique à 100% | 3,80% |
| - Triéthoxycaprylylsilane | 7,50% |
| - Méthylméthacrylate réticulé en billes à 100% | 3,00% |
| - Dioxyde de titane traité par des triméthoxycaprylylsilanes et des triéthoxycaprylylsilanes | 2,90% |
| - Fumarate de stéaryl sodium à 100% | 0,01% |
| - Parahydroxybenzoate de propyle | 0,15% |
| - Parahydroxybenzoate de méthyle | 0,25% |

formulation dans laquelle du mica et du talc sont toujours mélangés au triéthoxycaprylylsilane tandis que le parahydroxybenzoate de propyle et le parahydroxybenzoate de méthyle, commercialisés par la société Nipa sous la marque Nipasol M et respectivement sous la marque Nipagin M, interviennent au titre d'agents conservateurs.

Ces compositions solides sont inspirées d'une formulation conseillée par la société LCW - Les Colorants Wackherr, laquelle commercialise l'amidon carboxyméthylé sodique sous la marque Covagel, le mica mélangé à du triéthoxycaprylylsilane sous la marque Mica 8AS RO433 le talc mélangé à du triéthoxycaprylylsilane sous la marque Talc AS RO435, les billes de méthylméthacrylate réticulé sous la marque Covabead LH 85, le dioxyde de titane traité sous la marque PW Covasil S1 et enfin le fumarate de stéaryl sodium sous la marque Covafluid FS.

Il semble que l'amidon carboxyméthylé sodique de la composition solide (II) soit capable d'intégrer une quantité importante de la phase aqueuse (I). Ainsi, la phase aqueuse sera pour partie encapsulée au sein d'un premier support, l'agent gélifiant, et pour partie sera immobilisée à la surface d'un second support, le dioxyde de titane traité.

Il est évident que certaines des proportions indiquées ci-dessus peuvent varier légèrement, notamment la quantité d'eau de la phase liquide qui peut varier entre 50% et 90%, et de préférence entre 70% et 80%, ainsi que celles des deux agents actifs, à savoir l'agent dit "coup d'éclat", commercialisé sous la marque Eclaline 2, dont la quantité peut varier entre 1% et 4%, et l'acide ascorbique dont la quantité peut varier entre 0,05% et 0,20%.

On sait par ailleurs que le contour de l'oeil, zone bien précise du visage, présente des spécificités particulières. En effet, la peau du contour de l'oeil est constamment sollicitée, de l'ordre de 10.000 clignements par jour. De plus, la peau du contour de l'oeil est extrêmement fine, à savoir de 3 à 5 fois plus mince que la peau du visage.

En outre, la zone du contour de l'oeil est pauvre en graisse mais riche en muscles (22 au total, dont 14 s'activent toutes les 10 secondes).

Le relâchement cutané du contour de l'oeil est souvent le premier signe du vieillissement. Avec le temps, les fibres élastiques et les fibres de collagène s'altèrent, ce qui entraîne un relâchement et un flétrissement de la paupière inférieure, en suite de quoi une petite poche se forme.

La zone sensible sous les yeux, constamment sollicitée, laisse donc apparaître précocement des ridules, puis des rides dites d'expression prennent naissance pour former des pattes d'oie.

La peau plus fine du contour de l'oeil, voire sa transparence, rend plus visibles les soucis de circulation sanguine, d'où l'apparition d'ombres noires.

Avec l'âge, la fatigue et le stress, la microcirculation sanguine et lymphatique se ralentit, le creux de la paupière inférieure se colore, et tout ceci se traduit par l'apparition de cernes.

La présente invention propose donc également une composition spécifique pour le soin du contour des yeux, cette composition, toujours à base d'acide ascorbique à titre de premier agent actif, ayant pour propriétés de gommer les poches et les cernes sous les yeux.

Une telle composition est remarquable en ce qu'elle associe l'acide ascorbique à la caféine, autre produit actif dont on connait les propriétés stimulantes et drainantes ainsi que son activité sur la microcirculation.

De récentes études ont de surcroît montré que l'absorption de caféine accélère le phénomène d'apoptose. Il a donc été imaginé par la société déposante que la caféine en association avec l'acide ascorbique pourrait faciliter, par une sorte d'effet de synergie, l'élimination des cellules usées, voire mortes, de la peau ciblée au contour des yeux.

Une telle élimination a pu être vérifiée, et il est ainsi maintenant proposé un exemple général et un exemple préférentiel de composition cosmétique qui sont spécifiquement anti-poches et anti-cernes.

Exemples de Composition pour les soins du contour des yeux :

### A- Exemple général

### (I) Phase liquide aqueuse :

| | |
|---|---|
| - Eau osmosée | 50 à 90% |
| - Actifs hydrosolubles | 0 à 15% |
| - Acide ascorbique | 0,01 à 0,20% |
| - Caféine | 0,01 à 1% |
| - Triéthanolamine | 0,05 à 0,30% |
| - Conservateur(s) | 0 à 1% |

### (II) Poudre solide :

| | |
|---|---|
| - Agent(s) gélifiant(s) | 0,1 à 15% |
| - Emulsifiant(s) | 0 à 10% |
| - Actif(s) hydrosoluble(s) ou liposoluble(s) | 0 à 20% |
| - Particules traitées ou non en surface | 0 à 20% |
| - Liant(s) | 0 à 10% |
| - Conservateur(s) | 0 à 1% |

### B- Exemple préférentiel

| (I) Phase liquide aqueuse : | |
|---|---|
| - Eau osmosée | 73,699% |
| - Glycérine | 3,00% |
| - Polyoxyéthylène glycol | 5,50% |
| - Acide ascorbique | 0,02% |
| - Caféine pure | 0,01% |
| - Triéthanolamine à 100% | 0,06% |
| - Méthylisothiazolinone | 0,10% |
| - Colorant Blue 1 | 0,00028% |
| - Colorant Yellow 5 | 0,00034% |

### (II) Poudre solide :

| | |
|---|---|
| - Amidon carboxyméthylé sodique à 100% | 3,80% |
| - Triéthoxycaprylylsilane + mica | 2,40% |
| - Triéthoxycaprylylsilane + talc | 5,60% |
| - Méthylméthacrylate réticulé en billes à 100% | 3,00% |
| - Dioxyde de titane traité par des triméthoxycaprylylsilanes et des triéthoxycaprylylsilanes | 2,90% |
| - Fumarate de stéaryl sodium à 100% | 0,01% |
| - Parahydroxylbenzoate de propyle | 0,15% |
| - Parahydroxylbenzoate de méthyle | 0,25% |

Dans la composition détaillée ci-dessus, la caféine pure est celle commercialisée par la société BASF et les colorants Blue 1 et Yellow 5 sont distribués sous ces appellations par la société LCW-Les Colorants Wackherr déjà nommée.

Les compositions cosmétiques selon l'invention données à titre d'exemples préférentiels sont mises en oeuvre par le procédé suivant, étant ici précisé que la quantité de composition obtenue a été de 15 Kg.

On procède à un prémélange de la phase aqueuse (I) en introduisant toutefois seulement 80% de la quantité prévue de triéthanolamine.

On contrôle ensuite le pH du prémélange aqueux et on ajuste son pH en ajoutant lentement le reste de triéthanolamine jusqu'à parvenir impérativement à un pH compris entre 7,7 et 8,2.

Pour cela, dans les exemples précités, les 0,15%, 0,20% et respectivement 0,06% de triéthanolamine ont été consommés en totalité.

Il est clair que ces dernières quantités peuvent toutefois légèrement varier selon le pH de l'eau, auquel cas il est introduit soit un peu moins de triéthanolamine, soit un peu plus de cet ingrédient, le but étant de respecter rigoureusement la plage de pH de 7,7 à 8,2.

Le prémélange est effectué pendant quinze minutes jusqu'à parfaite homogénéité.

On forme par ailleurs la poudre solide (II) par mélange de tous ses ingrédients jusqu'à parfaite dispersion, laquelle a été obtenue en dix minutes.

Dans un mélangeur à hélices tripales ou équivalent, on ajoute ensuite la phase aqueuse (I) dans la phase solide (II) sous agitation modérée, à 1.500 tours par minute, pendant quinze minutes, temps au bout duquel on obtient une poudre homogène et fine conforme au standard préalablement défini.

En variante, la gélification de la phase aqueuse puis l'addition de la phase aqueuse à la phase solide permettant d'obtenir une composition sous forme de poudre homogène et fine, stable dans le temps, peut être réalisée dans un mélangeur avec agitateur de type batteur, équipé d'un variateur de vitesse allant jusqu'à 1.000 tours par minute. Le temps nécessaire à l'obtention d'une telle poudre est de 15 minutes à la vitesse de 600t/minute et de 10 minutes à la vitesse de 1.000t/minute.

Toutes les opérations précitées s'effectuent à température ambiante.

La poudre obtenue est non mouillante et elle peut donc être stockée et transportée sans précaution particulière, à la condition toutefois que la température environnante demeure comprise entre 8°C et 30°C.

Pour autant que cette plage de température soit respectée, la composition cosmétique en poudre selon l'invention peut être conservée en parfait état, avec tous ses principes actifs, jusqu'à dix-huit mois. Il a été par ailleurs vérifié qu'à la température constante de 40°C, la composition est toujours stable au bout de trois mois, et qu'il en est de même au bout de deux mois lorsque la composition a été maintenue en permanence à la température de 50°C. Il peut donc être affirmé que la composition selon l'invention est d'une stabilité certaine, alors pourtant qu'elle contient de l'acide ascorbique.

## Revendications

1. Composition cosmétique pour lutter contre le vieillissement de la peau, constituée et distribuée sous la forme d'une poudre se transformant en crème lors de son application, ladite composition étant du type de celles comprenant une phase aqueuse, qui est encapsulée dans ou immobilisée à la surface d'une poudre solide et qui est libérée sous l'effet d'une contrainte mécanique, ladite poudre solide comprenant un agent gélifiant de la phase aqueuse et des particules d'oxyde métallique présentant des propriétés de surface leur conférant une affinité faible vis-à-vis de ladite phase aqueuse, ladite composition étant **caractérisée en ce que** sa phase aqueuse comprend de l'acide ascorbique, à titre de premier agent actif, dans la plage en poids/poids de 0,01% à 0,20%, un second agent actif, choisi parmi une protéine végétale ou la caféine, et un agent d'ajustement de son pH entre 7,7 et 8,2.

2. Composition cosmétique selon la revendication 1, **caractérisée en ce que** sa phase aqueuse comprend de la triéthanolamine à titre d'agent d'ajustement de son pH.

3. Composition cosmétique selon la revendication 2, **caractérisée en ce que** le rapport en poids/poids de l'acide ascorbique et de la triéthanolamine varie entre environ 1:1,5 et environ 1:3.

4. Composition cosmétique selon l'une quelconque des revendications 1 à 3, **caractérisée en ce qu'**elle comprend en poids de 50 à 90% d'eau, et de préférence de 70% à 80% d'eau.

5. Composition cosmétique selon l'une quelconque des revendications 1 à 4, **caractérisée en ce que** le second agent actif contenu dans sa phase aqueuse est un agent actif dit "coup d'éclat", qui stimule le facteur de croissance vasculaire, ledit agent actif contenant de l'octénylsuccinate de protéine de lupin blanc doux hydrolysée.

6. Composition cosmétique selon la revendication 5, **caractérisée en ce que** l'octénylsuccinate de protéine de lupin blanc doux hydrolysée est contenu dans la composition dans la proportion de 0,30% à 0,90% en poids/poids.

7. Composition cosmétique selon l'une quelconque des revendications 1 à 6, **caractérisée en ce que** sa phase aqueuse comprend environ 1% de phénoxyéthanol.

8. Composition cosmétique selon l'une quelconque des revendications 1 à 4, **caractérisée en ce que** sa phase aqueuse comprend entre 0,01 et 1% de caféine pure.

9. Composition cosmétique selon l'une quelconque des revendications 1 à 8, **caractérisée en ce que** la poudre solide comprend de l'amidon carboxyméthylé sodique à titre d'agent gélifiant.

10. Composition cosmétique selon la revendication 9, **caractérisée en ce qu'**elle comprend entre 0,1 et 15% d'amidon carboxyméthylé sodique.

11. Composition cosmétique selon l'une quelconque des revendications 1 à 10, **caractérisée en ce que** les particules d'oxyde métallique contenues dans la poudre solide sont des particules de dioxyde de titane.

12. Composition cosmétique selon l'une quelconque des revendications 1 à 11, **caractérisée en ce que** sa phase aqueuse comprend environ 3% de glycérine.

13. Composition cosmétique selon l'une quelconque des revendications 1 à 12, **caractérisée en ce que** sa phase aqueuse comprend également au moins un additif cosmétique hydrosoluble choisi parmi les agents humectants, les agents solvants, les agents conservateurs et les agents colorants.

14. Composition cosmétique selon l'une quelconque des revendications 1 à 13, **caractérisée en ce que** sa phase poudre comprend également au moins un additif choisi parmi les agents liants et les agents conservateurs.

## Patentansprüche

1. Kosmetische Zusammensetzung zur Bekämpfung der Hautalterung, bestehend und vertrieben in der Form eines Puders, das sich bei seiner Anwendung in eine Creme wandelt, wobei diese Zusammensetzung von einer solchen Art ist, dass sie eine wässrige Phase aufweist, die eingekapselt ist in oder immobilisiert ist an der Oberfläche eines festen Puders und die unter der Wirkung einer mechanischen Beanspruchung freigegeben wird, wobei das feste Puder ein Geliermittel der wässrigen Phase und Metalloxidpartikel aufweist, die Eigenschafen der Oberfläche darstellen und ihr eine schwache Affinität gegenüber der wässrigen Phase verleiht, wobei die Zusammensetzung **dadurch gekennzeichnet ist, dass** seine wässrige Phase als erste aktive Substanz Ascorbinsäure in einem Bereich von Gewicht/Gewicht von 0,01% bis 0,20%, eine zweite aktive Substanz, die aus einem pflanzlichen Protein oder Koffein ausgewählt ist, und eine Substanz zum Einstellen ihres pH-Wertes zwischen 7,7 und 8,2 aufweist.

2. Kosmetische Zusammensetzung nach Anspruch 1, **dadurch gekennzeichnet, dass** seine wässrige Phase Triethanolamin als Substanz zum Einstellen ihres ph-Wertes aufweist.

3. Kosmetische Zusammensetzung nach Anspruch 2, **dadurch gekennzeichnet, dass** das Verhältnis in Gewicht/Gewicht der Ascorbinsäure und des Triethanolamin zwischen ungefähr 1:1,5 und ungefähr 1:3 variiert.

4. Kosmetische Zusammensetzung nach irgendeinem der Ansprüche 1 bis 3, **dadurch gekennzeichnet, dass** sie bezogen auf das Gewicht von 50 bis 90% Wasser und vorzugsweise von 70% bis 80% Wasser aufweist.

5. Kosmetische Zusammensetzung nach irgendeinem der Ansprüche 1 bis 4, **dadurch gekennzeichnet, dass** die zweite aktive Substanz, die in ihrer wässrigen Phase enthalten ist, eine aktive Substanz, ein sogenannter "Teintauffrischer" ist, der den vaskulären Wachstumsfaktor stimuliert, wobei die aktive Substanz Octenylsuccinat des hydrolisierten Proteins der weißen Süßlupine aufweist.

6. Kosmetische Zusammensetzung nach Anspruch 5, **dadurch gekennzeichnet, dass** das Octenylsuccinat des hydrolsierten Proteins der weißen Süßlupine in der Zusammensetzung im Verhältnis von 0,30% bis 0,90% bezüglich Gewicht/Gewicht enthaltend ist.

7. Kosmetische Zusammensetzung nach irgendeinem der Ansprüche 1 bis 6, **dadurch gekennzeichnet, dass** seine wässrige Phase ungefähr 1% Phenoxyethanol aufweist.

8. Kosmetische Zusammensetzung nach irgendeinem der Ansprüche 1 bis 4, **dadurch gekennzeichnet, dass** seine wässrige Phase zwischen 0,01 und 1% reinen Koffeins aufweist.

9. Kosmetische Zusammensetzung nach irgendeinem der Ansprüche 1 bis 8, **dadurch gekennzeichnet, dass** das feste Puder Carboxymethylstärke-Natrium als Geliermittel aufweist.

10. Kosmetische Zusammensetzung nach Anspruch 9, **dadurch gekennzeichnet, dass** sie zwischen 0,1 und 15% Carboxymethylstärke-Natrium aufweist.

11. Kosmetische Zusammensetzung nach irgendeinem der Ansprüche 1 bis 10, **dadurch gekennzeichnet, dass** die Metalloxidpartikel, die in dem festen Puder enthaltend sind, Titandioxidpartikel sind.

12. Kosmetische Zusammensetzung nach irgendeinem der Ansprüche 1 bis 11, **dadurch gekennzeichnet, dass** seine wässrige Phase ungefähr 3% Glyzerin aufweist.

13. Kosmetische Zusammensetzung nach irgendeinem der Ansprüche 1 bis 12, **dadurch gekennzeichnet, dass** seine wässrige Phase ebenfalls zumindest einen wasserlöslichen Kosmetikzusatz aufweist, der ausgewählt wird aus Feuchthaltemitteln, Lösungsmitteln, Konservierungsmitteln und Farbmitteln.

14. Kosmetische Zusammensetzung nach irgendeinem der Ansprüche 1 bis 13, **dadurch gekennzeichnet, dass** seine Puderphase ebenfalls zumindest einen Zusatz aufweist, der unter den Bindemitteln und den Konservierungsmitten ausgewählt wird.

## Claims

1. Cosmetic composition for combatting aging of the skin, which composition is formed and distributed in the form of a powder that transforms into a cream upon application, said composition being of the type comprising an aqueous phase which is encased in or immobilised on the surface of a solid powder and which is released under the effect of a mechanical stress, said solid powder comprising a gelling agent for the aqueous phase and metal oxide particles that have surface properties giving them a weak affinity to said aqueous phase, said composition being **characterised in that** its aqueous phase comprises ascorbic acid, as a first active agent, in a range of from 0.01 % to 0.20 % by weight, a second active agent selected from a vegetable protein or caffeine, and an agent for adjusting the pH of said aqueous phase to between 7.7 and 8.2.

2. Cosmetic composition according to claim 1, **characterised in that** the aqueous phase thereof comprises triethanolamine as the agent for adjusting its pH.

3. Cosmetic composition according to claim 2, **characterised in that** the ratio by weight of the ascorbic acid and of the triethanolamine varies between approximately 1:1.5 and approximately 1:3.

4. Cosmetic composition according to any of claims 1 to 3, **characterised in that** it comprises from 50 to 90 % by weight water, and preferably from 70 % to 80 % by weight water.

5. Cosmetic composition according to any of claims 1 to 4, **characterised in that** the second active agent contained in the aqueous phase thereof is an active agent referred to as "radiance-boosting" which stimulates the vascular growth factor, said active agent containing hydrolysed soft white lupin protein octenylsuccinate.

6. Cosmetic composition according to claim 5, **characterised in that** the hydrolysed soft white lupin protein octenylsuccinate is contained in the composition in the proportion of from 0.30 % to 0.90 % by weight.

7. Cosmetic composition according to any of claims 1 to 6, **characterised in that** its aqueous phase comprises approximately 1 % phenoxyethanol.

8. Cosmetic composition according to any of claims 1 to 4, **characterised in that** its aqueous phase comprises between 0.01 and 1 % pure caffeine.

9. Cosmetic composition according to any of claims 1 to 8, **characterised in that** the solid powder comprises sodium carboxymethylated starch as the gelling agent.

10. Cosmetic composition according to claim 9, **characterised in that** it comprises between 0.1 and 15 % sodium carboxymethylated starch.

11. Cosmetic composition according to any of claims 1 to 10, **characterised in that** the metal oxide particles contained in the solid powder are titanium dioxyde particles.

12. Cosmetic composition according to any of claims 1 to 11, **characterised in that** its aqueous phase comprises approximately 3 % glycerin.

13. Cosmetic composition according to any of claims 1 to 12, **characterised in that** its aqueous phase also comprises at least one water-soluble cosmetic additive selected from humectants, solvents, preservatives and colourants.

14. Cosmetic composition according to any of claims 1 to 13, **characterised in that** its powder phase also comprises at least one additive selected from binding agents and preservatives.
